# EUROPEAN PATENT SPECIFICATION

(11) **EP 3 226 931 B1**
(45) Date of publication and mention of the grant of the patent: **20.02.2019**
(21) Application number: 15804227.5
(22) Date of filing: 01.12.2015
(51) Int. Cl.: A61M 5/00, A61B 50/33, A61J 7/00

(54) **ANAESTHETIC CARRIER**
ANÄSTHETISCHE TRÄGER
SUPPORT D'ANESTHÉSIQUES

(30) Priority: 01.12.2014 GB 201421285
(43) Date of publication of application: 11.10.2017
(73) Proprietor: Uvamed Ltd., Loughborough, Leicestershire LE11 3PE (GB)
(72) Inventor: FAWDINGTON, Keith, Loughborough Leicestershire LE11 3PE (GB); GUPTA, Shashi, Loughborough Leicestershire LE11 3PE (GB)
(74) Representative: Browne, Robin Forsythe
(86) International application number: PCT/GB2015/053666
(87) International publication number: WO 2016/087841

(56) References cited:
- EP-A2- 0 254 487
- CH-A5- 584 631
- US-A1- 2005 101 905
- US-A1- 2010 012 537

## Description

This invention relates to a carrier for dosages of medicines, particularly but not exclusively to a carrier for syringes or ampoules containing dosages of drugs for use during surgical procedures, especially in operating theatres. The invention finds particular application for carrying several syringes or ampoules each containing one of a selection of drugs which may be required by an anaesthetist, registrar, nurse or other medical practitioner during a surgical procedure.

It is common in an operating theatre for syringes containing predetermined dosages of drugs to be prepared beforehand and provided in one or more trays arranged as required by the anaesthetist or other practitioner. A first tray may contain drugs for routine use and second and third trays may contain drugs for use in emergency situations. Mistakes are rare, but when they occur the consequences may be severe. Among the possible consequences, a patient may be paralysed but non-anaesthetised or a cardiac event may happen. Labelling of syringes is standardised, but syringes for different drugs, for example syntocinon and saline have the same coloured labels and may be of the same size. Furthermore, syringes for different drugs may be drawn by different practitioners. Protocols involving checking by a second person are used, but following them may not always be possible in an emergency. Such checking increases time pressure on the practitioner and may result in possible distraction of the practitioner. A second person may not be immediately available during an emergency. US2005101905 discloses a pharmaceutical dosage form carrier as described in the preamble of claim 1.

According to the present invention, as defined by claim 1, a pharmaceutical dosage form carrier comprises a base and an array of upwardly opening receptacles extending from the base, the receptacles being elongate and arranged in a side-by-side array wherein each receptacle comprises; a respective base region and one or more upwardly extending side walls; each receptacle being dimensioned to accommodate a container for a predetermined dosage of a specific drug; wherein each base region comprises a coloured display panel, the colour being selected to provide a visual indication of the predetermined dosage form in the container to be accommodated in the receptacle.

The container may comprise a syringe or ampoule containing the dosage form of the specific drug. The container may also carry a label which may be colour coded.

The receptacles may be arranged in a parallel array so that the carrier may comprise a generally rectangular configuration. Preferably the carrier is arranged so that each container for a dosage form may lie above a respective display panel. This arrangement enables colouring of the label of the dosage form to be viewed simultaneously with the panel.

The carrier is preferably formed from a unitary moulding which is preferably composed of transparent polymeric material. Use of transparent material allows the coloured panel to be observed from either side. A transparent material particularly allows the coloured panel to be observed simultaneously with the dosage forms to enable a user to confirm that the dosage forms are correctly located.

The carrier may comprise a transparent polymeric tray configured to receive one or a plurality of transparent inserts, the or each insert including one or more of said receptacles, wherein the tray may further comprise an array of coloured panels wherein each panel is arranged to be visible through the base of a transparent insert, or the base of each insert may comprise a respective coloured panel.

In a preferred embodiment the tray and insert are both composed of transparent polymeric material, preferably from moulded sheet material.

The insert may comprise an array of upwardly opening receptacles separated by upwardly extending ribs, a lower surface of the insert comprising rebates corresponding to the interior surfaces of the ribs;
the tray comprising a tray base with two or more upwardly extending locating ribs, each locating rib being configured to be received in a respective rebate on the lower surface of the insert.

The rectangular tray may have front and rear up-standing walls and upstanding side walls extending between the front and rear walls, wherein the length of the side walls is less than the length of the front and rear walls;
two locating ribs extending upwardly between the front and rear walls, parallel to the side walls;
wherein the distance between a first locating rib and an adjacent side wall is different to the distance of a second locating rib and the other side wall.

The plan view of the tray and locating ribs may be asymmetric so that the location of these ribs is different if the tray is rotated through 180°. The configuration of the upstanding extending ribs and corresponding rebates of the insert is also asymmetric so that the insert may be only placed into the tray in engagement with the locating ribs with the locating ribs received in the rebates in a single orientation.

This prevents the insert from being placed in the tray incorrectly.

The carrier may comprise a peripheral flange, a cover being arranged to engage the flange to provide a sealed package. The cover may be formed from a transparent rigid polymeric material to allow visual inspection and checking of the contents of the carrier. The carrier and cover may have an interlocking clip-on arrangement.

Alternatively a peelable film may be applied to the tray to form a tamper proof cover.

The carrier of this invention has particular application for carrying syringes containing dosage forms which may be required in a surgical procedure, for example, anaesthesia, chemotherapy, veterinary or dental procedures in which a specific range of drugs is required to be immediately to hand.

The carrier may also find particular application in a mobile surgical facility such as a military field hospital or a drop-in medical centre to which pre-packaged sets of syringes or ampoules may be transported and stored before use.

Sterilised packaging in accordance with this invention may comprise the carrier with an array of syringes or ampoules and a cover sealed in a sterile bag.

The containers are preferably syringes, each syringe containing a predetermined dosage form, the dosage forms being arranged in a clearly identifiable way.

Preferably the containers are configured to allow stacking with other containers.

The receptacles are preferably dimensioned so that each may accommodate a specific size of syringe, the receptacle having one or more of a length, width and depth selected to permit a practitioner to easily pick up the syringe in use while making it visually evident if an inappropriately sized syringe is located within the receptacle. This reduces the chances of a syringe being misplaced.

Preferably the receptacles are configured to allow stacking with other corresponding receptacles.

Syringes for surgical use may have labels with a predetermined colour system. Use of trays with colour coded bases in accordance with the present invention has the advantage that a mismatch with the label may be more easily detected without the need to read the label.

**Table 1 lists standard background colours for user applied syringe labels.**

| **Drug Class** | **Examples** | **Pantone® colour (uncoated)** |
|---|---|---|
| Induction agents | Thiopentone, etomidate, ketamine, propofol | Pantone®Yellow (process) |
| Hypnotics | Diazepam, lorazepam, midazolam | Pantone® 151 (orange) |
| Hypnotic antagonists | Flumazenil | Pantone® 151 (orange) with white diagonal stripes |
| Muscle relaxants | Succinylcholine, pancuronium, atracurium, mivacurium, rocuronium | Pantone® 805 (fluorescent red or warm red) |
| Relaxant antagonists | Neostigmine, edophonium, pyridostigmine | Pantone® 805 (fluorescent red or warm red) with white diagonal stripes |
| Narcotics | Morphine, fentanyl, remifentanil | Pantone® 297 (blue) |
| Narcotic antagonists | Naloxone | Pantone® 297 (blue) with white diagonal strips |
| Major tranquilizers | Droperidol, chlorpromazine | Pantone® 156 (salmon) |
| Vasopressors | Epinephrine, ephedrine, phenylephrine | Pantone® 256 (violet) |
| Hypotensive agents | Nitroprusside, nitroglycerine, phentolamine | Pantone® (violet) with white diagonal strips |
| Local anaesthetics | Lignocaine, bupivacaine | Pantone® 401 (grey) |
| Anticholinergic agents | Atropine, glycopyrrolate | Pantone® 367 (green) |
| Other agents | Oxytocin, heparin, protamine, antibiotics | Pantone® transparent white |

The carrier comprises a base tray and one or more of a plurality of inner trays, the base tray having one or more locations defined by formations configured to engage complementary formations of the or each respective inner tray of the array, the configuration of each location being arranged so that no other tray may be engaged at that location. Preferably a single insert is employed, the formations being configured so that the insert may be only engaged in the tray in a single orientation.

The base tray may have a double skin construction, comprising inner and outer polymeric sheets bonded at the upper periphery.

The base of this tray preferably comprises a peripheral downwardly extending projection flange or lip arranged to support the lower surface of the base above a work surface, the display being applied to the lower surface of the base. The lip preferably forms a continuous circumferential skirt extending around the base.

Provision of a projection flange or lip to raise the base of the tray above the work surface prevents abrasion or liquid damage to a coloured display provided on the lower surface of the tray. Multiple use of the base is facilitated.

A flange or lip may be provided around the upper edge of the carrier, inserts or both.

The display may be applied by printing or as a coloured adhesive film.

Alternatively a coloured printed sheet may be inserted between the skins to provide a waterproof display.

The display may additionally comprise labelling to provide a written indication of the correct intended dosage form of a syringe to be located in each receptacle.

The carrier may be formed by injection molding, thermoforming or by any other conventional means.

A carrier in accordance with this invention confers several advantages. A systematic approach to drawing up of drugs is facilitated allowing standardisation of storage of syringes, reducing any likelihood of confusion relating to location of the syringes in the carrier. Safe transfer of syringes between environments, for example the anaesthetic room and the theatre, is facilitated. Labelling may be visually compared to the coloured base so that misallocation of a syringe is more readily observed than when conventional fibreboard trays are employed.

The base may be colour coded in accordance with Royal College of Anaesthetists Guidelines. Enhanced visualisation of the printed black lettering on the syringe label is facilitated to emphasise when a syringe is placed in an incorrect section.

The invention is further described, by means of example but not in any limitative sense, with reference to the accompanying drawings, of which:

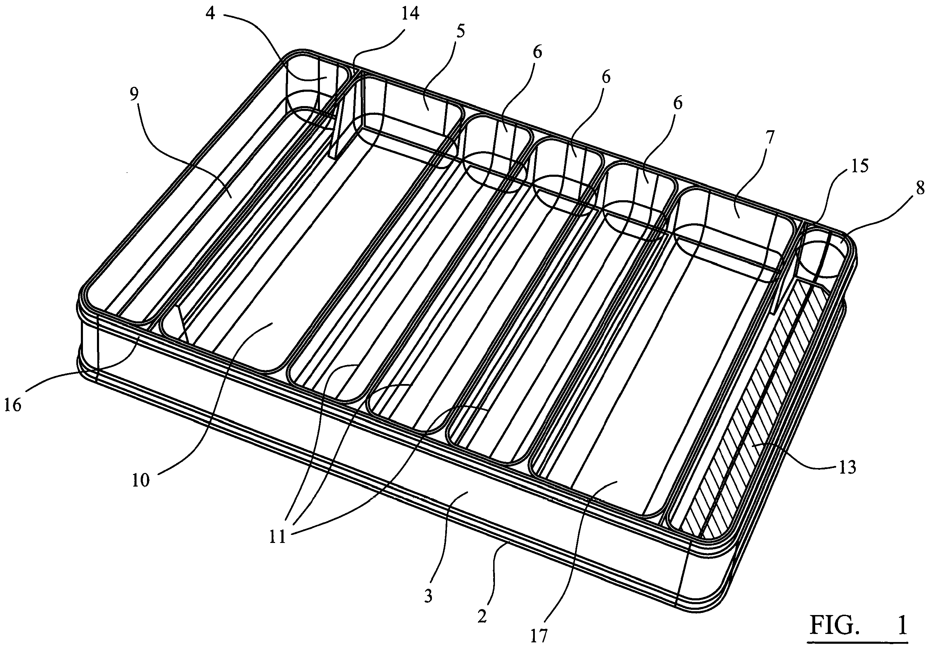

Figure 1 is a perspective view of a carrier in accordance with this invention;

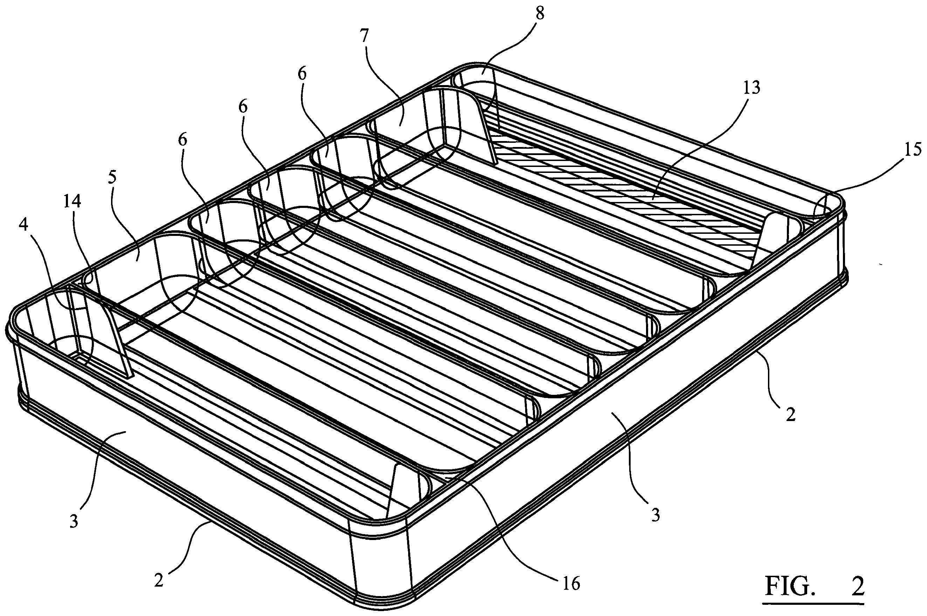

Figure 2 is a further perspective view of the carrier shown in Figure 1;

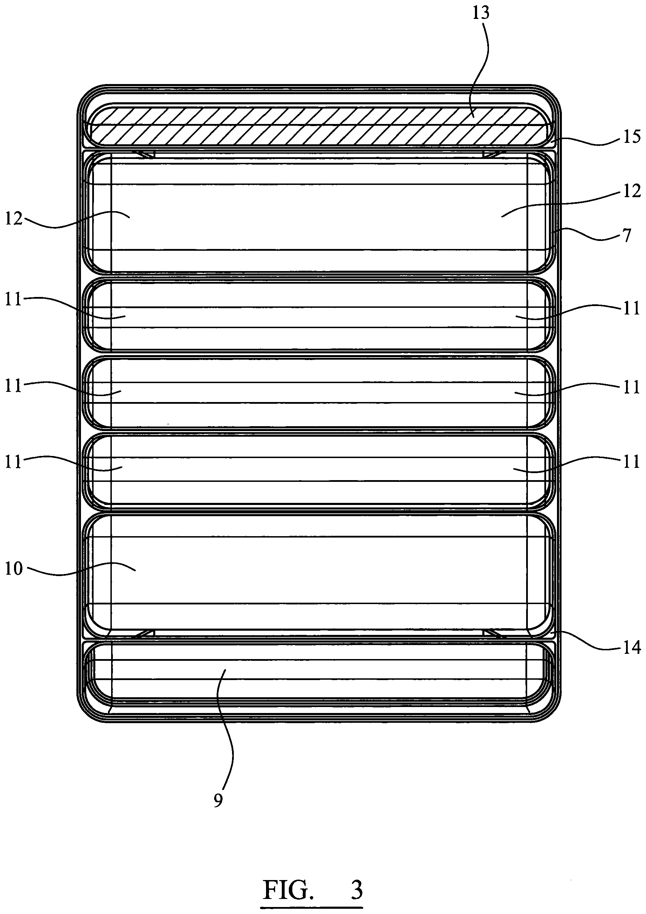

Figure 3 is a plan view of the carrier;

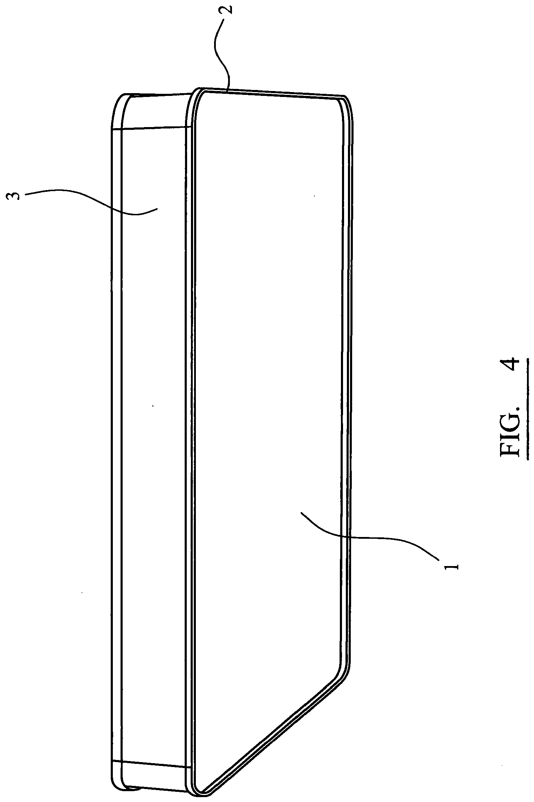

Figure 4 is a view of the carrier from below;

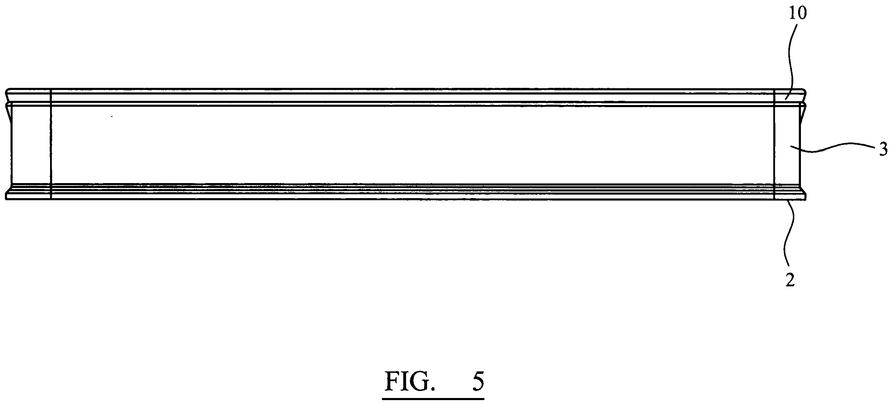

Figure 5 is a side elevation view of the carrier;
Figure 6 shows a side elevation and views from above and below of a large tray base;
Figure 7 shows an insert for the base shown in Figure 6;
Figure 8 shows a medium sized tray base;
Figure 9 shows the insert for the base shown in Figure 8;
Figure 10 shows a small tray base; and
Figure 11 shows an insert for the small tray base shown in Figure 10.

The carrier shown in the Figures shows a generally rectangular base (1) having a peripheral downwardly extending lip (2), upwardly extending top, bottom and side walls (3). The top, bottom and side walls and base define a generally rectangular cavity in which one or more of a plurality of insert trays are received. The cavity may be divided into sub-cavities by cross-members (14,15) extending between the top and bottom walls and which form locating ribs.

The insert trays are elongate and parallel-sided, having respective bases (9-13) and upwardly extending side walls (4-8). A first tray comprises a base (9) and side walls (4) with a width and depth selected to receive and accommodate a specifically dimensioned syringe without allowing excessive movement of the syringe during transportation.

A second tray has a base (10) and side walls (5) with a width and depth selected to accommodate two or more syringes containing a dosage of a preselected drug.

Third, fourth and fifth trays have a base (11) and side walls (6) with a width and depth to each accommodate a single syringe of a preselected drug.

A sixth tray has a base (12) and side walls (7) with a width and depth selected to accommodate a large diameter syringe containing a preselected drug or alternatively to accommodate two or more smaller syringes.

A seventh tray has a raised base (13) and side walls (8), the raised base (13) reduces the depth of the receptacle to allow a practitioner to easily pick up a syringe having a small diameter.

A peripheral engagement flange extends around the upper surface of the side walls (6) to engage a cover or lid (not shown). Preferably, the cover is formed of transparent polymeric material and is arranged to clip onto the flange.

The base (1) has an array of coloured display panels. Each panel has a length and width allowing it to be exclusively observed through the base of a single respective tray (9,10,11,12,13).

Each coloured display may have a single Pantone or a pattern of differently coloured stripes.

In a further embodiment (not shown) the wall at the proximal side of the tray may have an indent in order to receive and locate a syringe plunger.

The lower flange (2) preferably extends continuously around the entire lower periphery of the carrier integral with the base (1) to provide a skirt forming an entire lower surface of the carrier in order to facilitate cleaning and sterilisation and to prevent ingress of fluids beneath the carrier in use.

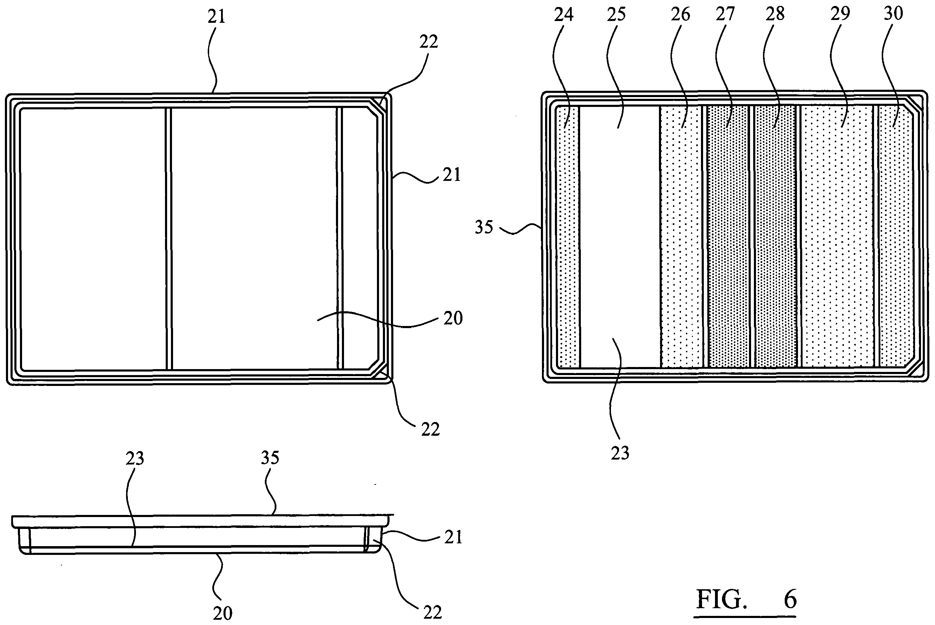

Figures 6 to 11 illustrate an alternative embodiment comprising a kit of large, medium and small carriers. Each carrier comprises a base with a tray insert configured to be received in only one orientation within the base.

Figure 6 shows plan views from below and above and a side elevation of a large dimensioned tray. The tray comprises a rectangular base panel (20) and a continuous side wall (21) having four rectangular sides. Adjacent corners (22) have inwardly extending triangular portions arranged to prevent the insert from being inserted into the tray in an incorrect orientation as described below.

In the upwardly facing interior surface (23) of the base is marked with seven parallel rectangular coloured panels (24 to 30). Each panel has a different colour formed by application of a coloured film, by printing or otherwise.

In a preferred example the first panel (24) is coloured orange, Pantone® 151 and may have a length of 40mm, width of 225mm and depth of 35mm to receive a 5ml capacity syringe.

The second panel (25) may be coloured Pantone® yellow having a length 60mm, width 225mm and depth 35mm to receive a 50ml syringe. The third panel (26) may be coloured Pantone® 805 fluorescent/warm red having a length of 40mm, width of 225mm and depth of 35mm to receive a 10ml capacity syringe body. The fourth panel (27) may be coloured Pantone® 297 blue and may have a length of 40mm, width of 225mm and depth of 35mm to receive a 50ml syringe body. The fifth panel (28) may be coloured Pantone® 156 salmon and have a length of 40mm, width 225mm and depth of 35mm to receive a 5ml syringe body. The sixth panel (29) may be coloured Pantone® transparent white and may have a length of 50mm, width 225mm and depth 35mm to receive a 20ml syringe body. The seventh panel (30) may be coloured Pantone® 805fluorescent/warm red with white diagonal stripes and have a length of 30mm, width 225mm and depth of 20mm to receive a 5ml syringe body. The coloured panels may include labels with written details of the dosage forms contained in the respective syringes.

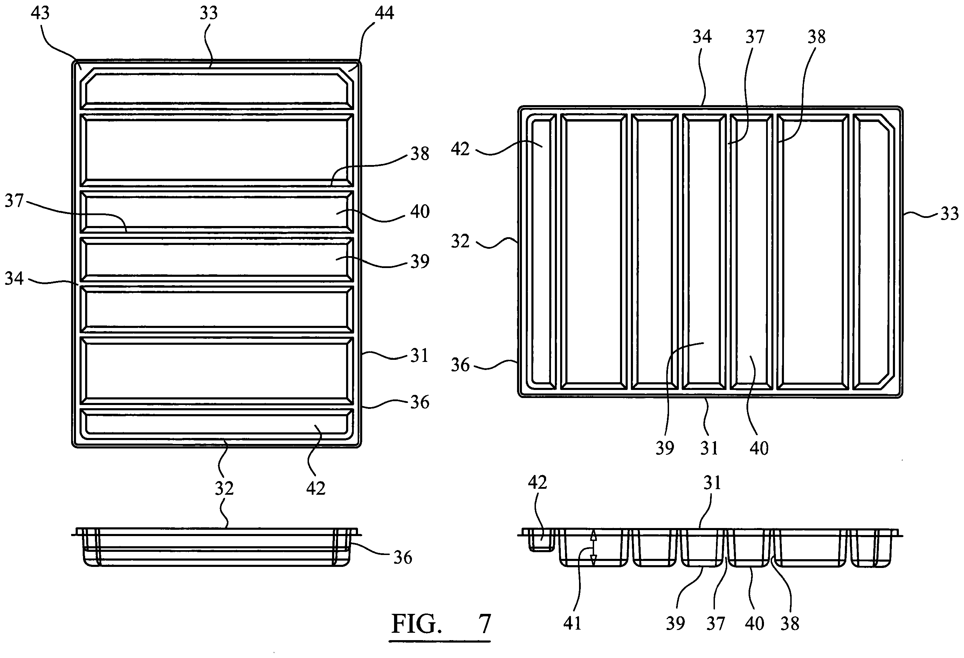

Figure 7 shows a transparent tray insert. The insert comprises an upper peripheral flange having sides (31) and (34) and ends (32, 33). The flange is arranged to engage and overlie the upper edge (35) of the side wall (21) of the base shown in Figure 6. The flange has a lip (36) which extends downwardly over the edge (35) of the base to prevent ingress of liquid or debris between the tray and insert during use.

An array of parallel generally rectangular receptacles formed by downwardly extending side walls e.g. (37, 38) and base portions e.g. (39,40) the depth (41) of each receptacle is the same for most receptacles so that the bases (39, 40) form a continuous support surface. However the first cavity (42) has a reduced depth to accommodate a smaller, for example 5ml syringe.

The coloured panels (24 to 30) of the tray base are visible through the transparent insert.

Adjacent corners (43, 44) at one end of the insert have triangular indented portions arranged to co-operate with complementary shaped dented portions (22) so that the insert may only be inserted into the tray base in one orientation preventing misalignment of the syringes with the respective coloured panels.

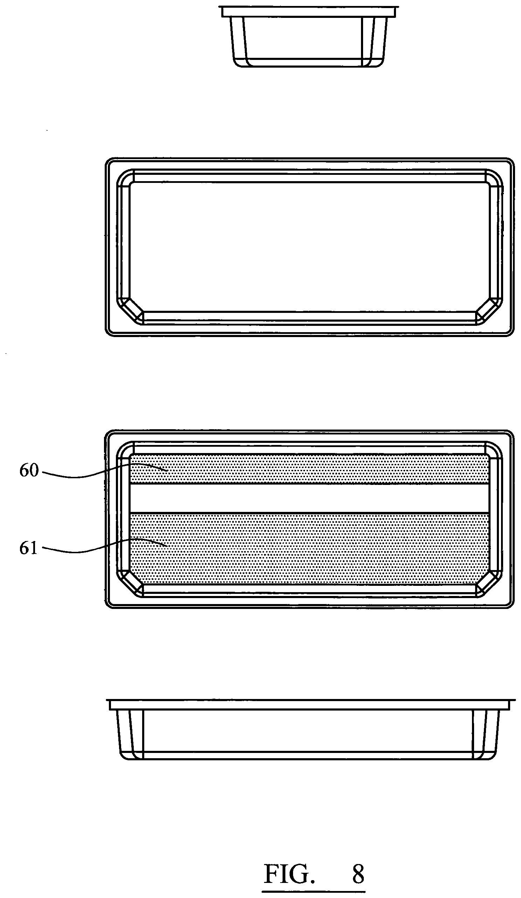

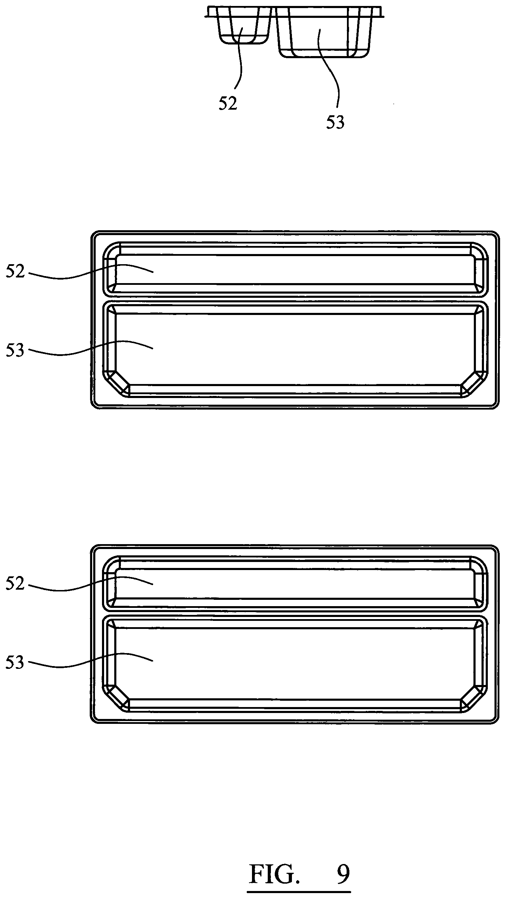

Figure 8 shows a base for a medium sized carrier, the general configuration is similar to that shown in Figure 6 except that the insert, shown in Figure 9 has two receptacles to receive two syringes.

The base has only rectangular coloured panels (50, 51) visible from above.

The insert shown in Figure 9 has two receptacles (52, 53) dimensioned to overlie the panels (50, 51) so that the panels may be observed through the basis of the transparent insert during use. The receptacle (52) is shallower in depth than the receptacle (53) so that a smaller sized syringe may be received and conveniently removed for use by a medical practitioner.

In a preferred example the first panel (52) is coloured violet, Pantone® 256 and may have a length of length of 55mm, width of 225mm and a depth of 35mm to receive a 50 ml syringe body. The second panel is coloured green, Pantone® 367 and may have a length of 35mm, width of 225mm and a depth of 20mm to receive a 5 ml syringe body.

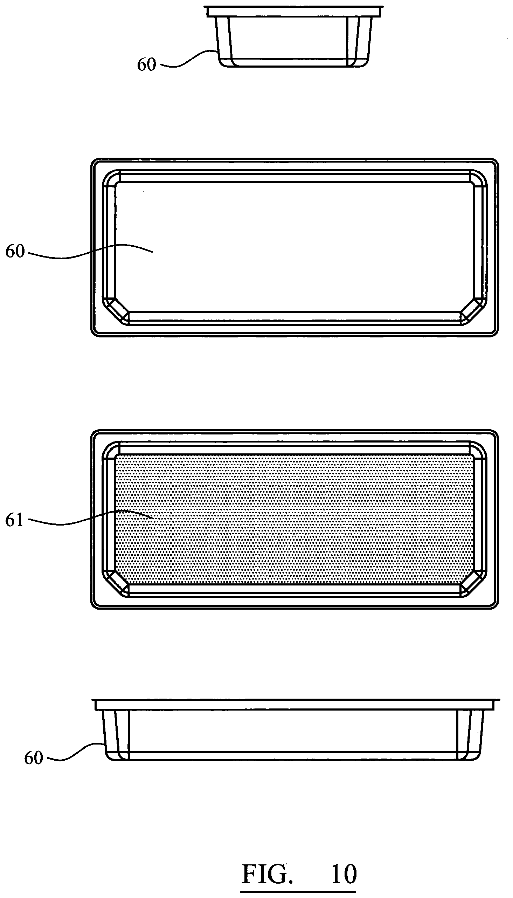

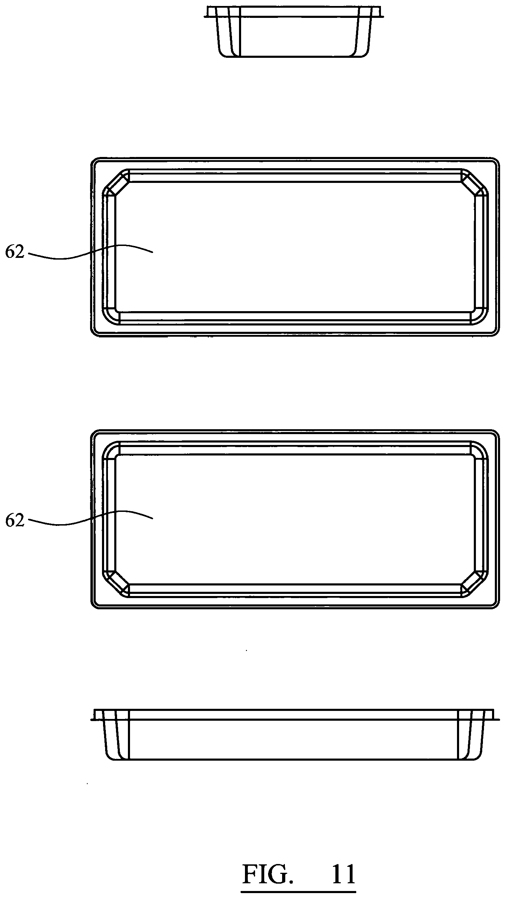

Figures 10 and 11 show a third smaller base (60) having a single coloured panel (61) visible through the base of the insert (62) shown in Figure 11.

In a preferred example the panel (61) is coloured grey, Pantone® 401 and may have a length of length of 110mm, width of 225mm and a depth of 35mm to receive a 50 ml syringe body

The carrier arrangement shown in Figures 6 to 11 may be provided individually or as a kit of two or three carriers as required by a practitioner.

## Claims

1. A pharmaceutical dosage form carrier comprising:
a base tray (1) having a base and side walls (3); and
one or more transparent inserts;
the base tray (1) being configured to receive the one or more transparent inserts;
each transparent insert having one or more upwardly opening receptacles extending from the base tray (1), the receptacles being elongate and arranged in a side-by-side array;
each receptacle comprising:
a respective base region (9-13); and
one or more upwardly extending side portions (4-8);
each receptacle being dimensioned to accommodate a container containing a predetermined dosage of a drug;
wherein the base tray (1) comprises one or more coloured panels arranged so that each coloured panel is visible through the base (9-13) of the transparent inserts, the colour being selected to provide a visual indication of the dosage form to be accommodated in the receptacle;
**characterised in that** the base tray (1) has one or more locations defined by formations configured to engage complementary formations on the one or more transparent inserts, the formations being arranged so that the transparent inserts are receivable within the base tray (1) in only one configuration.

2. A carrier as claimed in claim 1, wherein the base tray (1) comprises an upper and lower tray; wherein the upper tray has a complementary shape to the lower tray to allow housing of the upper tray within the lower tray and wherein the upper and lower trays may be peripherally bonded together.

3. A carrier as claimed in claim 2, wherein the one or more coloured panels are sealed between the upper and lower tray.

4. A carrier as claimed in any preceding claim, wherein the one or more coloured panels are detachable from the base tray (1).

5. A carrier as claimed in any preceding claim, wherein the complementary formations restrict lateral movement of the transparent inserts.

6. A carrier as claimed in any preceding claim, wherein at least one of the formations of the base tray (1) is a circumferentially extending shelf.

7. A carrier as claimed in any preceding claim, wherein the receptacles are arranged in a parallel array.

8. A carrier as claimed in any preceding claim, wherein each container lies above the coloured panels in use.

9. A carrier as claimed in any preceding claim, wherein the dosage forms are syringes or ampoules.

10. A carrier as claimed in any preceding claim, comprising a unitary moulding of opaque polymeric material.

11. A carrier as claimed in any preceding claim, wherein the transparent inserts have a peripheral flange arranged to engage a sealing cover.

12. A carrier as claimed in claim 11, wherein the base tray (1) has a peripheral flange to contact the perimeter of the peripheral flange of the transparent inserts.

13. A carrier as claimed in any preceding claim, wherein each receptacle is dimensioned to accommodate a specific size of syringe.

14. A carrier as claimed in any preceding claim, wherein the base comprises a peripheral downwardly extending projection flange arranged to support the lower surface of the base above a work surface, the display being applied to the lower surface of the base.

15. A dosage form kit comprising a carrier as claimed in any preceding claim, an array of syringes and a cover sealed within a sterile package.

## Patentansprüche

1. Träger für pharmazeutische Darreichungsformen, der Folgendes aufweist:
- eine Grundschale (1) mit einer Grundfläche und Seitenwänden (3); und
- einen oder mehrere transparente Einsätze;
- wobei die Grundschale (1) so konfiguriert ist, dass sie den einen oder die mehreren transparenten Einsätze aufnimmt;
- wobei jeder transparente Einsatz ein oder mehrere nach oben offene Behältnisse aufweist, die sich von der Grundschale (1) aus erstrecken, wobei die Behältnisse langgestreckt und in einer nebeneinanderliegenden Anordnung angeordnet sind;
- wobei jedes Behältnis Folgendes aufweist:
- einen jeweiligen Basisbereich (9 - 13); und
- einen oder mehrere sich nach oben erstreckende Seitenbereiche (4 - 8);
- wobei jedes Behältnis so dimensioniert ist, dass es einen Behälter aufnimmt, der eine vorbestimmte Dosis eines Arzneimittels enthält;
wobei die Grundschale (1) eine oder mehrere farbige Tafeln aufweist, die so angeordnet sind, dass die jeweilige farbige Tafel durch die Basis (9 - 13) der transparenten Einsätze sichtbar ist, wobei die Farbe so ausgewählt ist, dass sie eine visuelle Anzeige der in dem Behältnis aufzunehmenden Darreichungsform bietet;
**dadurch gekennzeichnet,**
**dass** die Grundschale (1) eine oder mehrere Stellen aufweist, die durch Ausformungen gebildet sind, die so konfiguriert sind, dass sie mit komplementären Ausformungen an dem einen oder den mehreren transparenten Einsätzen in Eingriff kommen, wobei die Ausformungen so angeordnet sind, dass die transparenten Einsätze in der Grundschale (1) in nur einer Konfiguration aufgenommen werden können.

2. Träger nach Anspruch 1,
wobei die Grundschale (1) eine obere Schale und eine untere Schale aufweist; wobei die obere Schale eine komplementäre Form zu der unteren Schale aufweist, um die Aufnahme der oberen Schale innerhalb der unteren Schale zu ermöglichen, und wobei die obere Schale und die untere Schale am Umfang miteinander verbunden werden können.

3. Träger nach Anspruch 2,
wobei die eine oder die mehreren farbigen Tafeln zwischen der oberen und unteren Schale eingesiegelt sind.

4. Träger nach einem der vorhergehenden Ansprüche,
wobei die eine oder die mehreren farbigen Tafeln von der Grundschale (1) abnehmbar sind.

5. Träger nach einem der vorhergehenden Ansprüche,
wobei die komplementären Ausformungen eine seitliche Bewegung der transparenten Einsätze beschränken.

6. Träger nach einem der vorhergehenden Ansprüche,
wobei mindestens eine der Ausformungen der Grundschale (1) ein sich in Umfangsrichtung erstreckender Einschub ist.

7. Träger nach einem der vorhergehenden Ansprüche,
wobei die Behältnisse in einer parallelen Anordnung angeordnet sind.

8. Träger nach einem der vorhergehenden Ansprüche,
wobei jeder Behälter im Gebrauch oberhalb der farbigen Tafeln liegt.

9. Träger nach einem der vorhergehenden Ansprüche,
wobei die Darreichungsformen Spritzen oder Ampullen sind.

10. Träger nach einem der vorhergehenden Ansprüche,
der ein einheitliches Formteil aus einem opaken Polymermaterial aufweist.

11. Träger nach einem der vorhergehenden Ansprüche,
wobei die transparenten Einsätze einen Umfangsflansch aufweisen, der so angeordnet ist, dass er mit einem Verschlussdeckel in Eingriff kommt.

12. Träger nach Anspruch 11,
wobei die Grundschalc (1) einen Umfangsflansch aufweist, um mit dem Umfang des Umfangsflansches der transparenten Einsätze in Kontakt zu kommen.

13. Träger nach einem der vorhergehenden Ansprüche,
wobei jedes Behältnis so dimensioniert ist, dass es eine bestimmte Größe von Spritzen darin unterbringen kann.

14. Träger nach einem der vorhergehenden Ansprüche,
wobei die Grundfläche einen sich nach unten erstreckenden Umfangsflansch aufweist, der so angeordnet ist, dass er die untere Fläche der Grundfläche oberhalb einer Arbeitsoberfläche abstützt, wobei ein Display an der unteren Fläche der Grundfläche angebracht ist.

15. Darreichungsform-Kit, der Folgendes aufweist: einen Träger nach einem der vorhergehenden Ansprüche, eine Anordnung von Spritzen und eine Abdeckung, die innerhalb einer sterilen Verpackung eingesiegelt sind.

## Revendications

1. Support de formes posologiques pharmaceutiques comprenant :
un plateau de base (1) présentant une base et des parois latérales (3) ; et
un ou plusieurs inserts transparents ;
le plateau de base (1) étant configuré pour recevoir l'insert transparent ou les inserts transparents ;
chaque insert transparent présentant un ou plusieurs récipients s'étendant depuis le plateau de base (1) et s'ouvrant vers le haut, les récipients étant de forme allongée et disposées en un réseau côte-à-côte ;
chaque récipient comprenant :
une zone de base respective (9-13) ; et
une ou plusieurs parties latérales s'étendant vers le haut (4-8) ;
chaque récipient étant dimensionné pour pouvoir loger un conteneur contenant une dose prédéterminée de médicament ;
selon lequel le plateau de base (1) comprend un ou plusieurs panneaux colorés, disposés de sorte que chaque panneau coloré est visible à travers la base (9-13) des inserts transparents, la couleur étant choisie pour fournir un indicateur visuel de la forme posologique devant être logée dans le récipient ;
**caractérisé en ce que** :
le plateau de base (1) présente un ou plusieurs endroits définis par des formes configurées pour s'engager des formes complémentaires présentes sur les inserts transparents, les formes étant disposées de sorte que les inserts transparents ne peuvent être reçus dans le plateau de base (1) que selon une seule configuration.

2. Support selon la revendication 1, selon lequel le plateau de base (1) comprend un plateau supérieur et un plateau inférieur ;
selon lequel le plateau supérieur présente une forme complémentaire au plateau inférieur afin de permettre au plateau supérieur d'être logé dans le plateau inférieur et selon lequel les plateaux supérieur et inférieur peuvent être collés en périphérie l'un à l'autre.

3. Support selon la revendication 2, selon lequel le ou les panneaux colorés sont enfermés entre le plateau supérieur et le plateau inférieur.

4. Support selon l'une quelconque des revendications précédentes, selon lequel le ou les panneaux colorés sont détachables du plateau de base (1).

5. Support selon l'une quelconque des revendications précédentes, selon lequel les formes complémentaires limitent tout mouvement latéral des inserts transparents.

6. Support selon l'une quelconque des revendications précédentes, selon lequel au moins une des formes du plateau de base (1) est une étagère qui s'étend de manière circonférentielle.

7. Support selon l'une quelconque des revendications précédentes, selon lequel les récipients sont disposés en un réseau parallèle.

8. Support selon l'une quelconque des revendications précédentes, selon lequel chaque conteneur est disposé au-dessus des panneaux colorés en utilisation.

9. Support selon l'une quelconque des revendications précédentes, selon lequel les formes posologiques sont des seringues ou des ampoules.

10. Support selon l'une quelconque des revendications précédentes, comprenant un matériau polymère opaque unitaire moulé.

11. Support selon l'une quelconque des revendications précédentes, selon lequel les inserts transparents présentent une bride périphérique disposée pour s'engager avec un couvercle de fermeture étanche.

12. Support selon la revendication 11, selon lequel le plateau de base (1) présente une bride périphérique se mettant au contact du périmètre de la bride périphérique des inserts transparents.

13. Support selon l'une quelconque des revendications précédentes, selon lequel chaque récipient est dimensionné de manière à recevoir une dimension spécifique de seringue.

14. Support selon l'une quelconque des revendications précédentes, selon lequel la base comprend une bride de projection périphérique s'étendant vers le bas disposée pour supporter la surface inférieure de la base au-dessus d'une surface de travail, l'affichage étant appliqué à la surface inférieure de la base.

15. Kit de forme posologique comprenant un support selon l'une quelconque des revendications précédentes, un réseau de seringues, et un couvercle, enfermés dans une conditionnement stérile.
